# EUROPEAN PATENT APPLICATION

(11) **EP 4 657 126 A1**
(43) Date of publication of application: **03.12.2025**
(21) Application number: 23918675.2
(22) Date of filing: 26.12.2023
(51) Int. Cl.: G02B 5/20, C09K 11/62, C09K 11/80, G01J 3/10, G01N 21/01, G01N 21/359

(54) **NEAR-INFRARED LIGHT-EMITTING DEVICE, SPECTROSCOPY APPARATUS, AND SPECTROSCOPY**

(30) Priority: 27.01.2023 JP 2023010889
(71) Applicant: Panasonic Intellectual Property Management Co., Ltd., Kadoma-shi, Osaka 571-0057 (JP)
(72) Inventor: NITTA, Mitsuru, Osaka 571-0057 (JP); SHIGITANI, Ryosuke, Osaka 571-0057 (JP); ABE, Takeshi, Osaka 571-0057 (JP); IWATA, Koki, Osaka 571-0057 (JP); FUJIWARA, Chigusa, Osaka 571-0057 (JP); OSHIO, Shozo, Osaka 571-0057 (JP)
(74) Representative: Müller-Boré & Partner Patentanwälte PartG mbB
(86) International application number: PCT/JP2023/046759
(87) International publication number: WO 2024/157721

(57) **Abstract**

In a near-infrared light emitting device (100), a spectral distribution of output light (20) has a light component derived from fluorescent light of a phosphor at least in a wavelength range of 600 nm or more and less than 980 nm, a center of gravity of a spectral distribution of wavelength converted light by the phosphor is in a wavelength range of 700 nm or more and less than 900 nm, a spectral intensity in a wavelength range of 660 nm or more and less than 900 nm has a minimum value that is more than 50% of a maximum value, in the spectral distribution, a light intensity of the spectral distribution of the wavelength converted light gradually decreases as a wavelength becomes shorter from a wavelength of 600 nm, and the light intensity gradually decreases as the wavelength becomes longer from a wavelength of 980 nm.

## Description

### TECHNICAL FIELD

The present invention relates to a near-infrared light emitting device, a spectroscopic apparatus, and a spectroscopy.

### BACKGROUND ART

A near-infrared light emitting device which simultaneously emits a visible component and a near-infrared component has been known in the past. The near-infrared light emitting device in the past is designed with consideration such that an irradiated object can be evaluated with both sensitivity characteristics of a detector and human's visual sense. The near-infrared light emitting device in the past enables the evaluation of the quality of an object to be evaluated with both machine eyes and human eyes. The near-infrared light emitting device in the past plays a role of supporting the detection of a foreign matter with machine eyes, which is often overlooked if human eyes alone are used.

### CITATION LIST

### PATENT LITERATURE

Patent Literature 1: WO 2022/176596

### SUMMARY OF INVENTION

However, there has been a problem that it is difficult for the near-infrared light emitting device in the past to evaluate the determination factor of the market value of a product which is an object to be inspected, although the near-infrared light emitting device in the past can perform rough evaluation such as foreign matter inspection with human eyes and machine eyes. For example, there has been a problem that it is difficult for the near-infrared light emitting device in the past to evaluate the determination factor of the market value such as presence or absence of the decay, sugar content, acidity, ripeness, and water content of objects to be inspected such as fruits and vegetables. Therefore, there has been a demand for improvement of a near-infrared light emitting device in the past to enable favorable evaluation of the factor.

The present invention has been devised in view of the problem of the prior art. An object of the present invention is to provide a near-infrared light emitting device which is suitable for evaluating the determination factor of the market value of an object to be inspected, and a spectroscopic apparatus and a spectroscopy using the near-infrared light emitting device.

In order to solve the above problem, a near-infrared light emitting device according to an aspect of the present invention emits output light and includes: a solid-state light emitting element; and a wavelength converter that includes a phosphor. A spectral distribution of the output light has a light component derived from fluorescent light of the phosphor at least in a wavelength range of 600 nm or more and less than 980 nm. A center of gravity of a spectral distribution of wavelength converted light by the phosphor is in a wavelength range of 700 nm or more and less than 900 nm. The center of gravity is a wavelength at which, an integrated value obtained by integrating a light intensity of a spectral distribution in increments of 1 nm of the wavelength converted light from a wavelength of 525 nm to a longer wavelength side, and an integrated value obtained by integrating the light intensity from a wavelength of 1100 nm to a shorter wavelength side, are equal. A spectral intensity in a wavelength range of 660 nm or more and less than 900 nm has a minimum value that is more than 50% of a maximum value. In the spectral distribution, the light intensity of the spectral distribution of the wavelength converted light gradually decreases as a wavelength becomes shorter from a wavelength of 600 nm.

A spectroscopic apparatus according to an aspect of the present invention includes the near-infrared light emitting device and irradiates an object to be inspected with the output light emitted by the near-infrared light emitting device.

A spectroscopy according to an aspect of the present invention uses the near-infrared light emitting device.

### BRIEF DESCRIPTION OF DRAWINGS

[FIG. 1] FIG. 1 is a diagram showing an example of a near-infrared light emitting device according to one embodiment.
[FIG. 2] FIG. 2 is a diagram showing an example of the spectral distribution of output light output by a near-infrared light emitting device according to Example 1.
[FIG. 3] FIG. 3 shows the spectral distribution obtained by adding an integrated value I_{S}, an integrated value I_{L}, and the center of gravity to the spectral distribution shown in FIG. 2.
[FIG. 4] FIG. 4 is a diagram showing an example of a near-infrared light emitting device according to another embodiment.
[FIG. 5] FIG. 5 is a diagram showing an example of a near-infrared light emitting device according to another embodiment.
[FIG. 6] FIG. 6 is a schematic diagram showing an example of a spectroscopic apparatus according to one embodiment.
[FIG. 7] FIG. 7 is a schematic diagram showing an example of a spectroscopic apparatus according to another embodiment.
[FIG. 8] FIG. 8 is a diagram showing an example of the spectral distribution of output light output by a near-infrared light emitting device according to Example 2.

### DESCRIPTION OF EMBODIMENTS

A near-infrared light emitting device, a spectroscopic apparatus, and a spectroscopy according to embodiments herein will be described in detail below with reference to the drawings. The dimensional proportions of the drawings are exaggerated for illustrative purposes and may differ from the actual proportions.

### [Near-infrared light emitting device]

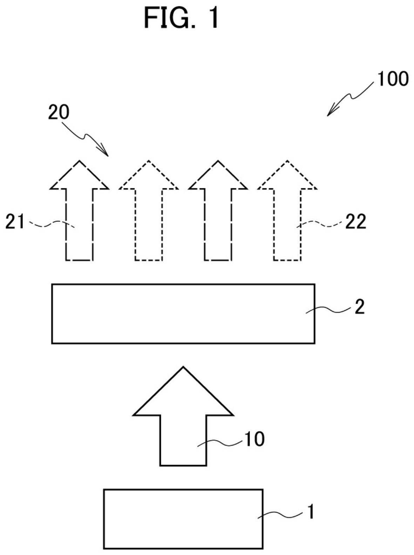

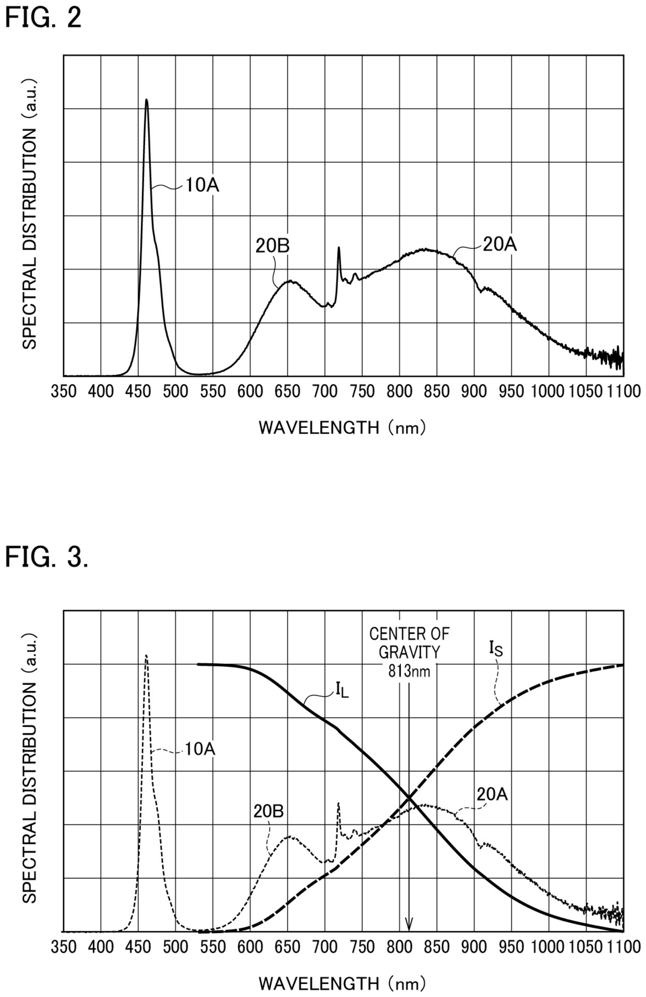

First, a near-infrared light emitting device 100 according to the present embodiment will be described. FIG. 1 is a diagram showing an example of the near-infrared light emitting device according to one embodiment. As shown in FIG. 1, the near-infrared light emitting device 100 includes a solid-state light emitting element 1 and a wavelength converter 2. The near-infrared light emitting device 100 is configured to emit output light 20. FIG. 2 is a diagram showing an example of the spectral distribution of the output light 20 output by the near-infrared light emitting device 100 according to one embodiment. Unless otherwise stated, in the present specification, fluorescent light and the output light 20 are emitted when the temperature of a phosphor or the wavelength converter 2 is 30°C.

The solid-state light emitting element 1 converts electricity into light, and when power is supplied thereto and the solid-state light emitting element 1 is driven, the solid-state light emitting element 1 converts the supplied electric energy into light energy. Then, the converted light energy is emitted from a light extraction surface as the output light 20. Typical examples of the solid-state light emitting element 1 include a light emitting diode (LED) and a laser diode (LD).

The wavelength converter 2 is a wavelength conversion element, and after receiving light, the wavelength converter 2 converts the light into light with a longer wavelength than the received light. That is, the wavelength converter 2 receives high-energy photons, converts the high-energy photons into low-energy photons, and outputs the low-energy photons. The wavelength converter 2 includes a phosphor. The phosphor may include an inorganic phosphor in which a fluorescent ion is added to an inorganic compound. The wavelength converter 2 may be a resin fluorescent film, a fluorescent ceramic, a composite, or the like. The composite may include at least one of a resin fluorescent film and a fluorescent ceramic. The wavelength converter 2 may include an inorganic compound in an amount of 95% by mass or more, in an amount of 99% by mass or more, or in an amount of 100% by mass. The wavelength converter 2 with the high ratio of inorganic compounds has excellent thermal conductivity, and therefore it is possible to enhance heat dissipation.

The resin fluorescent film may have particulate phosphors dispersed in a resin. The resin fluorescent film can be formed by curing a phosphor paste, which is obtained by mixing a resin and powdered phosphors, for example. As the resin, a translucent resin can be used, and an example of the resin is a silicone resin. The fluorescent ceramic may be a molded product formed by molding phosphors. The fluorescent ceramic can be formed by applying pressure, heating, and reaction sintering phosphor raw materials or phosphor powders, for example.

The near-infrared light emitting device 100 may be configured such that primary light 10 emitted by the solid-state light emitting element 1 thereof is used to irradiate at least the wavelength converter 2. Due to this kind of configuration, a phosphor included in the wavelength converter 2 absorbs the primary light 10, and converts the primary light 10 into light with a longer wavelength than the primary light 10. Then, wavelength converted light, which is the light with a longer wavelength that has been subjected to wavelength conversion, is emitted from the wavelength converter 2. Further, the primary light 10 which has not been absorbed by the phosphor passes through the wavelength converter 2, and then is emitted from the wavelength converter 2, together with the wavelength converted light. The primary light 10 and the wavelength converted light, which are emitted from the wavelength converter 2, are output as light components constituting the output light 20 emitted from the near-infrared light emitting device 100.

The wavelength converter 2 and the solid-state light emitting element 1 may be arranged with a gap therebetween, or the wavelength converter 2 may be arranged in contact with the solid-state light emitting element 1. When the wavelength converter 2 is arranged in contact with the solid-state light emitting element 1, the heat generated by the wavelength converter 2 can be thermally conducted through the solid-state light emitting element 1.

The spectral distribution of the wavelength converted light can be changed depending on the type and combination of phosphors. Further, it is possible to change the intensity ratio between the primary light 10 and the wavelength converted light emitted from the wavelength converter 2 by changing the concentration of a phosphor included in the wavelength converter 2 or the thickness of the wavelength converter 2. The wavelength converter 2 may include a visible phosphor and a near-infrared phosphor.

The visible phosphor emits visible fluorescent light 22 having a maximum fluorescence intensity in a wavelength range of visible light. The visible phosphor may be at least one phosphor selected from the group consisting of a green phosphor which emits light having a maximum fluorescence intensity in a wavelength range of 490 nm or more and less than 570 nm, a yellow phosphor which emits light having a maximum fluorescence intensity in a wavelength range of 570 nm or more and less than 585 nm, an orange phosphor which emits light having a maximum fluorescence intensity in a wavelength range of 585 nm or more and less than 620 nm, and a red phosphor which emits light having a maximum fluorescence intensity in a wavelength range of 620 nm or more and less than 780 nm. This can adjust the spectral distribution of a visible fluorescence component. Therefore, it is possible to adjust a visible fluorescence component of the output light 20 emitted from the near-infrared light emitting device 100 according to a usage application.

The visible phosphor may be at least one phosphor selected from the group consisting of a Ce³⁺ activated garnet phosphor, Eu²⁺ activated alkaline earth metal nitride silicate, and Eu²⁺ activated alkaline earth metal nitride aluminosilicate. These visible phosphors have high application achievements as phosphors for LED lighting. Therefore, procurement of these visible phosphors is easy, and additionally reliability and handling of these visible phosphors are assured. Therefore, it is possible to provide the near-infrared light emitting device 100 having these kinds of visible phosphors.

Examples of the Ce³⁺ activated garnet phosphor include a phosphor represented by a general formula RE₃Al₂(AlOn)₃:Ce³⁺, Ca₃Sc₂(SiO₄)₃:Ce³⁺, Y₃Ga₂(AlO₄)₃:Ce³⁺, and solid solutions thereof. In the above general formula, RE refers to a rare earth element, and is at least one rare earth selected from the group consisting of Sc, Y, La, Tb, Gd, and Lu, for example. Specific examples of the phosphor represented by the general formula RE₃Al₂(AlO₄)₃:Ce³⁺ include Lu₃Al₂(AlO₄)₃:Ce³⁺, Y₃Al₂(AlO₄)₃:Ce³⁺(YAG), and (Y, Gd₃Al₂(AlO₄)₃:Ce³⁺.

Examples of the Eu²⁺ activated alkaline earth metal nitride silicate include Sr₂Si₅N₈:Eu²⁺, Ca₂Si₅N₈:Eu²⁺, and solid solutions thereof.

An example of the Eu²⁺ activated alkaline earth metal nitride aluminosilicate is a phosphor represented by a general formula MAlSiN₃:Eu²⁺. In the general formula, M refers to magnesium or an alkaline earth metal, and is at least one metal element selected from the group consisting of Mg, Ca, Sr, and Ba, for example. Specific examples of the Eu²⁺ activated alkaline earth metal nitride aluminosilicate include CaAlSiN₃:Eu²⁺(CASN), SrAlSiN₃:Eu²⁺, (Sr, Ca)AlSiN₃:Eu²⁺(SCASN), and solid solutions thereof.

From thereamong, it is preferable that the visible phosphor contains at least one phosphor selected from the group consisting of YAG, CASN, and SCASN.

The near-infrared phosphor emits near-infrared fluorescent light 21 having a maximum fluorescence intensity in a wavelength range of near-infrared light. When the near-infrared phosphor is irradiated with excitation light, the near-infrared phosphor emits the near-infrared fluorescent light 21 having a maximum fluorescence intensity in a wavelength range of near-infrared light. The near-infrared fluorescent light 21 may have a spectral intensity at least over an entire wavelength range of 700 nm or more and 1000 nm or less. This kind of spectral distribution is suitable for detecting characteristic absorption bands of N-H stretching vibration, C-H stretching vibration, and O-H stretching vibration by means of a spectroscopy.

The near-infrared fluorescent light 21 may have a maximum fluorescence intensity in a wavelength range of 800 nm or more and less than 900 nm. This kind of near-infrared fluorescent light 21 can be emitted by using a LiGa₅O₈:Cr³⁺ near-infrared phosphor and a near-infrared phosphor similar to this, for example. Therefore, the wavelength converter 2 is configured by combining the near-infrared phosphors with visible phosphors with low temperature quenching, such as a Ce³⁺ activated garnet phosphor for LED lighting and an Eu²⁺ activated alkaline earth metal nitride aluminosilicate phosphor for LED lighting, and it is possible to provide the near-infrared light emitting device 100 having an advantageous form, for example. The near-infrared fluorescent light 21 preferably has a maximum fluorescence intensity in a wavelength range of 800 nm or more and 860 nm or less.

It is preferable that the half width of a spectrum with a maximum fluorescence intensity of the near-infrared fluorescent light 21 is more than 180 nm. Therefore, even if one kind of near-infrared phosphor is used, and several kinds of near-infrared phosphors are not used, it is possible to obtain a near-infrared component having the spectral distribution over a wide wavelength range of 700 to 1000 nm.

The near-infrared phosphor may have at least a chromium ion which functions as a fluorescent ion and which is added to a crystal of an inorganic compound with a spinel crystal structure that is the same as a spinel crystal structure of a compound LiGa₅O₈. Whether the near-infrared phosphor has a spinel crystal structure which is the same as a spinel crystal structure of the compound LiGa₅O₈ can be determined by measuring an X-ray diffraction pattern by means of an X-ray diffraction method. Specifically, this means that an XRD pattern of the near-infrared phosphor is substantially the same as an XRD pattern of the compound LiGa₅O₈ (registration number: 33716) registered in an ICSD.

The crystal may contain an alkali metal of a group 1 element, at least one of a group 13 element and scandium, and oxygen. That is, when the near-infrared phosphor is represented by a chemical formula A(D₁₋ₓCrₓ)₅O₅, A may mainly contain an alkali metal, and D may mainly contain at least one of a group 13 element and scandium. D may mainly contain a group 13 element.

A mainly containing an alkali metal means that A contains an alkali metal in an amount of 75 mol% or more. A may contain an alkali metal in an amount of 90 mol% or more, in an amount of 95 mol% or more, or in an amount of 100 mol%. Further, D mainly containing at least one of a group 13 element and scandium means that D contains at least one of a group 13 element and scandium in an amount of 75 mol% or more. D may contain at least one of a group 13 element and scandium in an amount of 90 mol% or more, in an amount of 95 mol% or more, or in an amount of 100 mol%.

The alkali metal is preferably at least one element selected from the group consisting of Li, Na, K, Rb, and Cs, and more preferably at least one of Li and K. The group 13 element is preferably at least one element selected from the group consisting of Al, Ga, and In, and more preferably Ga.

It is preferable that the amount of the alkali metal of the above crystal is small relative to a stoichiometric composition. In other words, it is preferable that the crystal has a composition in which an element A is deficient relative to a stoichiometric composition of A(D₁₋ₓCrₓ)₅O₈. In this way, a crystal with chemical properties similar to those of LiGa₅O₈ can be used as the near-infrared phosphor. Therefore, the near-infrared phosphor is expected to have unique fluorescence characteristics similar to fluorescence characteristics of the LiGa₅O₈:Cr³⁺ near-infrared phosphor. Furthermore, when application is made to spectroscopy, high-precision non-destructive measurement can be expected.

The near-infrared phosphor is represented by a general formula A_{y}(D₁₋ₓCrₓ)₅O₈. A mainly contains an alkali metal, D mainly contains at least one of a group 13 element and scandium, and y is preferably less than 1. When y is less than 1, it is possible to obtain a near-infrared phosphor in which the ratio of light emitting components of emission lines is small. The y is more preferably 0.90 or less, even more preferably 0.85 or less, and particularly preferably 0.83 or less. Further, y is preferably 0.70 or more. When y is 0.70 or more, it is less likely that the near-infrared phosphor and a β-Ga₂O₃:Cr³⁺ phosphor having a different phase are present in a mixed state, and it becomes easy to obtain desired unique fluorescence characteristics. The y is more preferably 0.75 or more, and even more preferably 0.77 or more.

The crystal mainly contains LiGa₅O₈, and it is preferable that a part of Ga is substituted with Cr. Here, "the crystal mainly containing LiGa₅O₈" means that the crystal contains LiGa₅O₈ in an amount of 75 mol% or more, and it is preferable that the crystal contains LiGa₅O₈ in an amount of 90 mol% or more.

A phosphor represented by a general formula LiGa₅O₈:Cr³⁺ (hereinafter referred to as LiGa₅O₈:Cr³⁺ phosphor) has been conventionally known as a deep red phosphor which emits a deep red fluorescence component of an emission line having a maximum fluorescence intensity in the vicinity of a wavelength of 715 to 720 nm. However, after experimental production of several LiGa₅O₈:Cr³⁺ phosphors and evaluation of fluorescence characteristics thereof, it was found that the LiGa₅O₈:Cr³⁺ phosphors function as near-infrared phosphors when the LiGa₅O₈:Cr³⁺ phosphors are experimentally produced under specific preparation and synthesis conditions. Further, it was also found that an example was present in which the LiGa₅O₈:Cr³⁺ phosphors exhibited the following unique fluorescence characteristic as the near-infrared phosphors.
(1) The phosphor has a maximum fluorescence intensity (fluorescence peak) in a wavelength range of 800 nm or more and 900 nm or less, such as the vicinity of a wavelength of 830 nm, for example.
(2) The phosphor has a fluorescence component over a wide wavelength range of 700 to 1000 nm.
(3) A fluorescent spectrum is very wide, and the half width of a spectrum having a maximum fluorescence intensity is more than 180 nm.
(4) The temperature quenching is very small. (A maximum fluorescence intensity at a phosphor temperature of 150°C, relative to a maximum fluorescence intensity at a phosphor temperature of 30°C is more than 90%)
(5) The wavelength conversion efficiency (internal quantum efficiency) for converting an absorbed visible component into a near-infrared component is more than 80%, even though the phosphors experimentally produced are in an early stage of development.

The near-infrared fluorescent light 21 or a near-infrared fluorescence component may have a spike in a wavelength range of 710 nm or more and 730 nm or less. This kind of spike is generally observed in a fluorescent spectrum of the LiGa₅O₈:Cr³⁺ near-infrared phosphor exhibiting unique fluorescence characteristics, although the reason is unclear. In other words, a near-infrared phosphor emitting fluorescent light with this kind of spike may have very small temperature quenching. Meanwhile, the near-infrared fluorescent light 21 or the near-infrared fluorescence component may not have the spike in a wavelength range of 710 nm or more and 730 nm or less. Alternatively, even if the near-infrared fluorescent light 21 or the near-infrared fluorescence component has the spike, when a maximum fluorescence intensity in a wavelength range of 800 nm or more and 860 nm or less is assumed to be I_{NIR}, and a maximum intensity of the spike is assumed to be I_{DR}, it is preferable that I_{DR} is less than two times of I_{NIR}. The spike refers to a sharp peak in the vicinity of a wavelength of 720 nm. When the spectral distribution is spectral data in increments of 1 nm, the spectral data of the spike changes by more than 7%/nm in the wavelength range, for example. The I_{DR} may be less than 1.25 times of I_{NIR}, less than 1.0 times of I_{NIR}, or less than 0.8 times of I_{NIR}.

As shown in FIG. 2, the spectral distribution of the output light 20 emitted from the near-infrared light emitting device 100 has a light component derived from fluorescent light of a phosphor at least in a wavelength range of 600 nm or more and less than 980 nm. Further, a spectral intensity in a wavelength range of 660 nm or more and less than 900 nm has the minimum value which is more than 50% of the maximum value. A spectral intensity in a wavelength range of 660 nm or more and less than 940 nm may have the minimum value which is more than 50% of the maximum value. It is preferable that the spectral distribution of the output light 20 has a light component derived from fluorescent light of a phosphor at least over an entire wavelength range of 600 nm or more and less than 980 nm.

As shown in FIG. 3, in the spectral distribution on the wavelength side longer than a wavelength of 525 nm, the center of gravity of the spectral distribution of wavelength converted light by a phosphor is in a wavelength range of 700 nm or more and less than 900 nm. The center of gravity is a wavelength at which an integrated value I_{S} and an integrated value I_{L} are equal. The integrated value I_{S} is obtained by integrating light intensities of the spectral distribution in increments of 1 nm of wavelength converted light from a wavelength of 525 nm to the longer wavelength side, and the integrated value I_{L} is obtained by integrating light intensities from a wavelength of 1100 nm to the shorter wavelength side. In the spectral distribution shown in FIG. 3, the center of gravity is a wavelength of 813 nm, and at the wavelength, the integrated value I_{S} and the integrated value I_{L} in FIG. 3 are substantially equal, for example. The expression that the integrated value I_{S} and the integrated value I_{L} are equal may mean that the integrated value I_{S} and the integrated value I_{L} are substantially equal within the margin of errors. Further, the center of gravity is preferably in a wavelength range of 750 nm or more and less than 850 nm, and more preferably in a wavelength range of 770 nm or more and less than 830 nm.

In a preferred form, the minimum value of a spectral intensity in a range of, a fluorescence peak wavelength of the solid-state light emitting element 1, or more and 900 nm or less, relative to maximum value of the spectral intensity, is less than 3%, and more preferably less than 1%. The minimum value of the spectral intensity is located in a wavelength range of, the fluorescence peak wavelength of the solid-state light emitting element 1, or more and 600 nm or less, particularly in a wavelength range of 500 nm or more and less than 550 nm. In the examples shown in FIGS. 2 and 3, the fluorescence peak wavelength of the solid-state light emitting element 1 is 460 nm. If the spectral distribution having this kind of light component is used, it is possible to suppress the output of light components which are not necessary for evaluating the quality of objects to be inspected such as fruits and vegetables, for example. Therefore, it is possible to provide the near-infrared light emitting device 100 which can efficiently use input power, which has excellent efficiency in converting electricity into light, and which is advantageous for achieving high output and high-precision quality evaluation thereby.

As shown in FIG. 3, in the spectral distribution, a light intensity of the spectral distribution of wavelength converted light may gradually decrease as a wavelength becomes shorter from a wavelength of 600 nm. That is, the output light 20 has the spectral distribution in which light components with intensities at or above a certain level are uniformly concentrated in red light components and near-infrared components with a wavelength of 600 nm or more. Therefore, the near-infrared light emitting device 100 can emit the output light 20 having the red light components and the near-infrared components with high efficiency. A light intensity may gradually decrease as a wavelength becomes longer from a wavelength of 980 nm. That is, the output light 20 has the spectral distribution in which light components with intensities at or above a certain level are uniformly concentrated in red light components and near-infrared components with a wavelength of 980 nm or less. Therefore, the near-infrared light emitting device 100 can emit the output light 20 having the red light components and the near-infrared components with high efficiency.

It is possible to apprehend the decay of an onion by means of a spectroscopy using a light component with a wavelength of 660 to 950 nm, it is possible to apprehend the ripeness and water content of a tomato by means of a spectroscopy using a light component with a wavelength of 620 to 950 nm, and it is possible to apprehend the sugar content and acidity of fruits and vegetables by means of a spectroscopy using a light component with a wavelength of 780 to 830 nm, for example. Therefore, if the spectral distribution having these kinds of light components is used, the near-infrared light emitting device 100 can emit the output light 20 which is suitable for quality evaluation for determining the market value of objects to be inspected such as fruits and vegetables, for example. Further, if the spectral distribution having these kinds of light components is used, it is possible to suppress the output of light components which are not necessary for evaluating the quality of objects to be inspected such as fruits and vegetables, for example. Therefore, it is possible to provide the near-infrared light emitting device 100 which can efficiently use input power, which has excellent efficiency in converting electricity into light, and which is advantageous for achieving high output and high-precision quality evaluation thereby.

A light component having the spectral distribution in a wavelength range of 600 nm or more and less than 980 nm may be a light component derived from the electron energy transition of at least one fluorescent ion of an Eu ion and a Cr ion. These ions emit red fluorescent light or near-infrared fluorescent light, which has a wide spectrum half width and has a relatively small intensity change relative to a wavelength. In addition, procurement and synthesis a red phosphor and a near-infrared phosphor containing these ions are easy. Therefore, by using an orthodox Eu ion-activated red phosphor and Cr ion-activated near-infrared phosphor, it is possible to obtain the output light 20 with a small intensity change in which light components are concentrated in a desired red to near-infrared wavelength region, and the near-infrared light emitting device is suitable for industrial production. The Eu ion may be Eu²⁺, for example. The Cr ion may be either Cr³⁺ or Cr⁴⁺, for example.

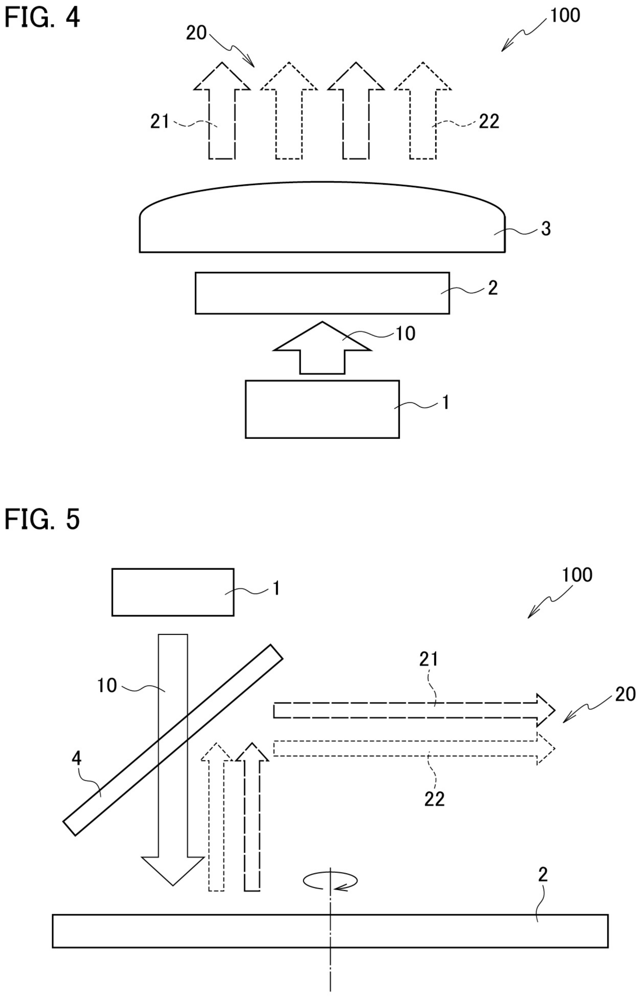

As shown in FIG. 4, the near-infrared light emitting device 100 may include a lens 3. The lens 3 may be a collimating lens. The lens 3 may be arranged such that wavelength converted light radiated from the wavelength converter 2 passes therethrough. The lens 3 may allow the output light 20 to pass therethrough, in addition to the wavelength converted light.

As shown in FIG. 4, the output light 20 may pass through the lens 3 and may be output, and the lens 3 may collimate the wavelength converted light radiated from the wavelength converter 2. In this form, the wavelength converted light radiated from the wavelength converter 2 as diffuse light is converted into collimated light (parallel light) and then is output. This is suitable for constructing an evaluation system for evaluating an object to be inspected by irradiating the object to be inspected with the output light 20 emitted from the near-infrared light emitting device 100 which is located far from the object to be inspected without bringing the near-infrared light emitting device 100 in close proximity to the object to be inspected. The distance between a surface of the object to be inspected which is an object to be irradiated and a light output unit of the near-infrared light emitting device 100 may be 1 cm or more and less than 1 m, 5 cm or more and less than 50 cm, or 10 cm or more and less than 50 cm.

As shown in FIG. 4, a light entering surface of the lens 3 may be arranged so as to cover an entirety of a light emission surface of the wavelength converter 2. Due to the configuration, almost all of wavelength converted light emitted from the wavelength converter 2 enters the light entering surface of the lens 3. This is suitable for converting light radiated from the wavelength converter 2 into collimated light. As shown in FIG. 4, the light entering surface of the lens 3 may have a plane portion and may be arranged such that wavelength converted light enters the plane portion. Further, the light emission surface of the wavelength converter 2 may also have a plane portion. The plane portion of the light entering surface of the lens 3 may be parallel to the plane portion of the light emission surface of the wavelength converter 2. The two plane portions may be arranged so as to be parallel and close to each other with a gap therebetween, or the two plane portions may be arranged so as to be in contact with each other.

It is also preferable that the wavelength converter 2 is arranged so as to be in surface contact with a principal light extraction surface of the solid-state light emitting element 1. This is because the heat generated by the wavelength converter 2 can be thermally conducted through the solid-state light emitting element 1. When both of the solid-state light emitting element 1 and the wavelength converter 2 are arranged to be in surface contact with each other, and the wavelength converter 2 and the lens 3 are arranged to be in surface contact with each other, the heat of the wavelength converter 2 can be thermally conducted to the lens 3 and dissipated, for example.

Although there are no particular limitations on the shape of the wavelength converter 2, the wavelength converter 2 may have a plate shape, for example. When the wavelength converter 2 has a plate shape, the thickness of the wavelength converter 2 may be 50 µm or more and less than 500 µm, for example. When the wavelength converter 2 has a thin-plate shape, the ratio of wavelength converted light leaked from a side surface of the wavelength converter 2 is reduced, and therefore the ratio of light entering the light entering surface of the lens 3 can be increased. This leads to a form which is advantageous in outputting high-quality coherent light. It is preferable that the thickness of the wavelength converter 2 is 80 µm or more and less than 300 µm.

The wavelength converter 2 may be disposed such that the wavelength converter 2 rotates. As shown in FIG. 5, the solid-state light emitting element 1 may emit the primary light 10, and the primary light 10 may be used to irradiate the rotating wavelength converter 2, for example. Due to the configuration, a position of an area of the wavelength converter 2 irradiated with the primary light 10 changes according to change in time. Therefore, the wavelength converter 2 of which the temperature rises due to being irradiated with the primary light 10 moves to a position where the wavelength converter 2 is not irradiated with the primary light 10, and the wavelength converter 2 is cooled down during the time. Therefore, even if the wavelength converter 2 is irradiated with the primary light 10 with a high energy density, the temperature rise of the wavelength converter 2 is suppressed, and the near-infrared light emitting device 100 can emit high output light 20. The wavelength converter 2 may be disposed on a rotatable rotation wheel.

FIG. 4 shows a form of the near-infrared light emitting device 100 which is referred to as a transmission type, and FIG. 5 shows a form of the near-infrared light emitting device 100 which is referred to as a reflection type. In the transmission type, the primary light 10 used to irradiate the wavelength converter 2 passes through the wavelength converter 2, and is output as the output light 20, together with wavelength converted light. In the reflection type, the primary light 10 used to irradiate the wavelength converter 2 is reflected by the wavelength converter 2 and is output as the output light 20 including wavelength converted light.

In the reflection type, it is preferable to use an optical element 4 shown in FIG. 5, which is referred to as a dichroic mirror and which has characteristics of allowing light with a specific wavelength to pass therethrough and reflecting light with other wavelengths. When the optical element 4 allows the primary light 10 to pass therethrough and reflects wavelength converted light, the primary light 10 can be used to irradiate the wavelength converter 2 efficiently and homogeneously, and additionally the wavelength converted light can be output at an angle in a direction perpendicular to an irradiation direction with the primary light 10. Therefore, it is possible to provide a range of options for the structural design of the near-infrared light emitting device 100.

### [Spectroscopic apparatus and spectroscopy]

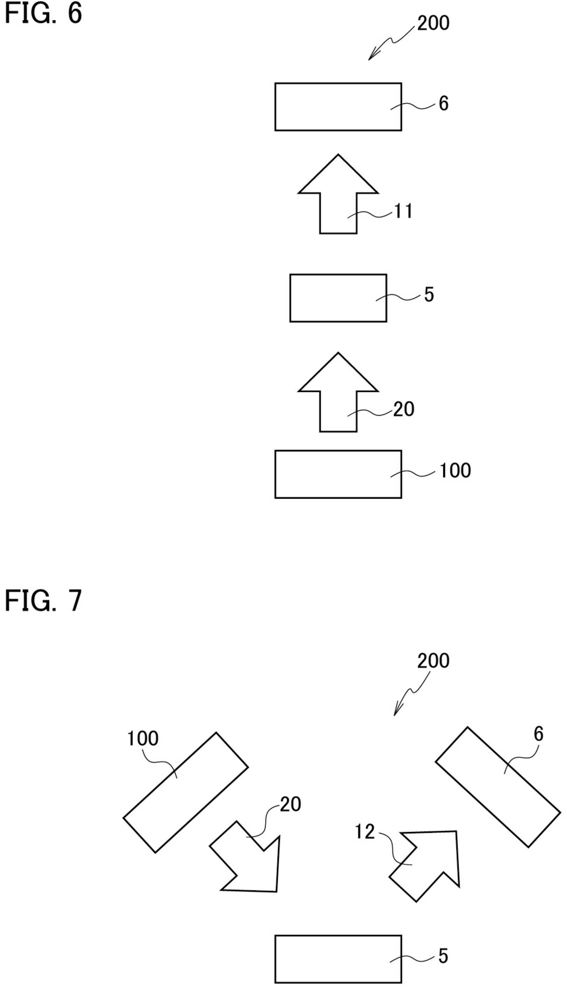

Next, with reference to FIGS. 6 and 7, a spectroscopic apparatus 200 and a spectroscopy according to the present embodiment will be described. The spectroscopic apparatus 200 is a near-infrared spectroscopic apparatus, for example. The spectroscopy is a near-infrared spectroscopy, for example. FIG. 6 is a schematic diagram showing an example of a transmission type spectroscopic apparatus 200. FIG. 7 is a schematic diagram showing an example of a reflection type spectroscopic apparatus 200. As shown in FIGS. 6 and 7, the spectroscopic apparatus 200 includes the near-infrared light emitting device 100. That is, the spectroscopic apparatus 200 relates to the spectroscopy using the near-infrared light emitting device 100. The spectroscopic apparatus 200 irradiates an object 5 to be inspected with the output light 20 emitted by the near-infrared light emitting device 100.

As shown in FIG. 6, the transmission type spectroscopic apparatus 200 includes the near-infrared light emitting device 100 and a spectroscope 6. The object 5 to be inspected is interposed between the near-infrared light emitting device 100 and the spectroscope 6. The near-infrared light emitting device 100 is configured to emit the output light 20. The object 5 to be inspected is arranged such that the object 5 to be inspected is irradiated with the output light 20. The spectroscope 6 is arranged to receive transmitted light 11, which has passed through the inside of the object 5 to be inspected, especially near-infrared transmitted light, out of the output light 20 used to irradiate the object 5 to be inspected. The spectroscope 6 detects and disperses the transmitted light 11. The spectroscope 6 may be a near-infrared spectroscope, for example. The spectroscopic apparatus 200 may be an inspection apparatus. The inspection apparatus may analyze spectral data dispersed by the spectroscope 6. The inspection apparatus may include an analysis unit (not shown), and may apprehend or determine whether inspection items such as the internal state and quality of the object 5 to be inspected are favorable using the analysis unit. The analysis unit may include a Central Processing Unit (CPU), a Read Only Memory (ROM), and a Random Access Memory (RAM). The CPU may read a program and reference data stored in the ROM, and perform information processing related to the analysis of the inspection items according to the program.

As shown in FIG. 7, the reflection type spectroscopic apparatus 200 also includes the near-infrared light emitting device 100 and the spectroscope 6. However, in the reflection type spectroscopic apparatus 200, the spectroscope 6 is arranged to receive reflected light 12, which has been reflected by the object 5 to be inspected, especially near-infrared reflected light, out of the output light 20 used to irradiate the object 5 to be inspected. Configurations other than the above of the reflection type spectroscopic apparatus 200 are similar to those of the transmission type spectroscopic apparatus 200, and therefore description thereof will be omitted.

The reflection type spectroscopic apparatus 200 may include a reflective member (not shown) such as a metal plate, particularly a reflective member for near-infrared light. Suppose that a near-infrared fluorescence component of the output light 20, which has passed through the object 5 to be inspected, is reflected by the reflective member, and again passes through the inside of the object 5 to be inspected. In the above case, even if the object 5 to be inspected is a liquid or the like, it is possible to obtain near-infrared spectral data specific to the object 5 to be inspected without difficulty.

The transmitted light 11 and the reflected light 12 may have at least near-infrared fluorescence components, and may have red fluorescence components. Depending on a form of the object 5 to be inspected, a visible fluorescence component can be either transmitted or reflected. In a form in which a visible component is reflected, the visible component may be dispersed, or may be used for a visual inspection of the object 5 to be inspected.

The object 5 to be inspected may be food, for example. The term "food" is a general term for articles eaten by humans, such as lunch boxes ingredients, grains, fruits and vegetables, meat, fish, processed foods, and beverages.

The usage application of the spectroscopic apparatus 200 is not particularly limited. The object 5 to be inspected may be fruits and vegetables, or may be foods other than fruits and vegetables, such as a general term for articles eaten by humans including lunch boxes ingredients, grains, meat, fish, processed foods, and beverages, for example. The spectroscopic apparatus 200 may be an apparatus for evaluating objects to be inspected such as fruits and vegetables, for example. Since the spectroscopic apparatus 200 irradiates fruits and vegetables with light suitable for evaluating, the decay, ripeness, sugar content, acidity, and water content of the fruits and vegetables, the spectroscopic apparatus 200 is advantageous for the precise inspections for determining the market value of objects to be inspected such as fruits and vegetables, for example. The spectroscopic apparatus 200 may also be used as a foreign matter inspection apparatus for inspecting whether the object 5 to be inspected contains a foreign matter.

As described above, the spectroscopic apparatus 200 includes the near-infrared light emitting device 100. Further, the spectroscopy uses the near-infrared light emitting device 100. Then, an object to be inspected is irradiated with the output light 20 emitted by the near-infrared light emitting device 100. Light components are concentrated in red light components and near-infrared components of the near-infrared light emitting device 100 in a wavelength range of 600 nm or more and less than 980 nm, more preferably in a wavelength range of 660 nm or more and less than 940 nm, and even more preferably in a wavelength range of 660 nm or more and less than 900 nm. Moreover, light components with relatively small intensity change are used to irradiate an object to be inspected. It is possible to apprehend the decay of an onion by means of a spectroscopy using a light component with a wavelength of 660 to 950 nm, it is possible to apprehend the ripeness and water content of a tomato by means of a spectroscopy using a light component with a wavelength of 620 to 950 nm, and it is possible to apprehend the sugar content and acidity of fruits and vegetables by means of a spectroscopy using a light component with a wavelength in the vicinity of 780 to 830 nm, for example. Therefore, it is possible to provide the spectroscopic apparatus 200 and the spectroscopy suitable for high-precision evaluation of objects to be inspected such as fruits and vegetables. Here, fruits and vegetables are sometimes collectively referred to as vegetables.

In the above embodiment, spectrums of the spectral distribution of the output light 20, the near-infrared fluorescent light 21, and the visible fluorescent light 22 may be spectrums when a wavelength of the primary light 10 (excitation light) is 450 nm, unless otherwise stated. Further, the spectrums may be spectrums at 30°C, unless otherwise stated. Specifically, the spectrums may be spectrums when the temperature of a phosphor or the wavelength converter is 30°C.

### [Examples]

The present embodiment will be described in additional detail below by means of examples and comparative examples, but the present embodiment is not limited to these examples. The evaluation was performed at 30°C, unless otherwise stated.

Three kinds of self-produced near-infrared phosphors were used in a near-infrared light emitting device of examples. The synthesis of the near-infrared phosphors and evaluation results of phosphors will be described below. First, common points of synthesis and evaluation of the near-infrared phosphors will be explained, and then evaluation results of the synthesized near-infrared phosphors will be explained.

### (Preparation of phosphor raw materials)

The following compound powders were used as raw materials for self-produced near-infrared phosphors.
- Lithium carbonate (Li₂CO₃): purity 2N, manufactured by FUJIFILM Wako Pure Chemical Corporation
- Gallium oxide (Ga₂O₃): purity 4N, manufactured by Nippon Rare Metal, Inc.
- Gadolinium oxide (Gd₂O₃): purity 4N, manufactured by Shin-Etsu Chemical Co., Ltd.
- Scandium oxide (Sc₂O₃): purity 4N, manufactured by Shin-Etsu Chemical Co., Ltd.
- Chromium oxide (Cr₂O₃): purity 3N, manufactured by Kojundo Chemical Laboratory Co., Ltd.

### (Preparation and synthesis of phosphors)

Oxide phosphors were synthesized by means of a preparation method using a solid phase reaction. Specifically, three kinds of near-infrared phosphors with the following preparation compositions were synthesized.
1) LGO:Li(Ga_{0.97}Cr_{0.03})₅O₈
2) GGG:Gd₃(Ga_{0.925}Cr_{0.075})₂(GaO₄)₃
3) GSO:(Ga_{0.59}Sc_{0.40}Cr_{0.01})₂O₃

The above raw materials were weighed and prepared to achieve the above preparation composition, that is, to achieve the ratio of metal elements of the above preparation composition. In the ratio of metal elements of the above preparation composition of the LGO, the ratio of atoms Li : Ga : Cr was 1.00 : 4.85 : 0.15, for example. The preparation raw materials were wet-mixed using a planetary ball mill (manufactured by FRITSCH GmbH, Germany), and dry powders thereof were used as mixed raw materials. The above mixed raw materials were put in an alumina crucible with a lid, and calcined in the atmosphere or weakly reducing atmosphere for four hours using a box-type electric furnace. The calcination temperature of the LGO was 1500°C, the calcination temperature of the GGG was 1600°C, and the calcination temperature of the GSO was 1400°C. A calcined product obtained by calcining was lightly crushed and used as a phosphor sample.

### (Evaluation of crystal of phosphor)

X-ray diffraction patterns of compounds constituting a phosphor sample were examined using an X-ray diffraction device (manufactured by Rigaku Corporation.). All of the X-ray diffraction patterns were mainly composed of a desired compound (LiGa₅O₈, Gd₃Ga₂(GaO₄)₃, or (Ga, Sc)₂O₃). From the result, it was found that a phosphor which used, as a main phase, a crystal phase that was substantially the same as that of LiGa₅O₈, Gd₃Ga₂(GaO₄)₃, or (Ga, Sc)₂O₃ was synthesized.

A composition of a phosphor sample of the LGO was examined using an inductively coupled plasma optical emission spectrometer (manufactured by Thermo Fisher Scientific K.K.). It was found that the composition was Li_{0.81}(Ga_{0.75}Cr_{0.03})₅O_{8-α}, and the composition was a Li-poor composition in which Li was deficient by 15 atom% or more (less than 30%), relative to the preparation composition (Li(Ga_{0.97}Cr_{0.03})₅O₈).

### (Evaluation of fluorescence characteristics)

Although details are omitted, the following are results obtained by measuring or evaluating fluorescence characteristics of each phosphor when excited at an excitation wavelength of 450 nm using an absolute PL quantum yield spectrometer (manufactured by Hamamatsu Photonics K.K.). An FWHM is the half width of a spectrum having a fluorescence peak wavelength (maximum fluorescence intensity).
1) LGO:
   Fluorescence peak wavelength: 833 nm
   Fluorescent spectrum shape: broad
   FWHM: 207 nm
   Internal quantum efficiency: 85%
   Light absorption rate: 63%
2) GGG:
   Fluorescence peak wavelength: 731 nm
   Fluorescent spectrum shape: broad
   FWHM: 96 nm
   Internal quantum efficiency: > 95%
   Light absorption rate: 48%
3) GSO:
   Fluorescence peak wavelength: 823 nm
   Fluorescent spectrum shape: broad
   FWHM: 147 nm
   Internal quantum efficiency: 92%
   Light absorption rate: 41%

In the present example, a self-produced near-infrared phosphor and a commercially available red phosphor were used in this way.

### (Example 1)

A near-infrared light emitting device (wavelength conversion type light emitting element) having a solid-state light emitting element and a wavelength converter shown in FIG. 1 was fabricated using the following members 1) to 4).
1) Solid-state light emitting element
   High-power blue LED (actual peak wavelength: 460 nm) (product number: SMBB450H-1100, Ushio Inc.)
2) Wavelength converter
   2-1) CaAlSiN₃:Eu²⁺ (CASN) red phosphor (fluorescence peak wavelength: 651 nm) (Mitsubishi Chemical Corporation)
   2-2) LiGa₅O₈:Cr³⁺ (LGO) near-infrared phosphor (fluorescence peak wavelength: 833 nm) (fabricated by means of the method described above)
   2-3) Two-liquid mixing type of thermosetting silicone resin (sealing material for LED, product name: KER-2600A/B, Shin-Etsu Chemical Co., Ltd.)

### (Fabrication of wavelength converter)

A wavelength converter was fabricated by laminating a first wavelength converter (one CASN red phosphor sheet) and a second wavelength converter (two LGO near-infrared phosphor sheets) which were fabricated as follows.

### (Fabrication of first wavelength converter)

0.68 g of a CASN red phosphor and a silicone resin (1 g of KER-2600A, 1 g of KER-2600B) were mixed using a stirring defoaming device, and then further defoamed. The stirring defoaming device used at this time was manufactured by THINKY CORPORATION, the product name was AWATORI RENTARO (registered trademark), and the model was ARE-310. Further, the rotational speed of the stirring defoaming device was approximately 2000 rpm, and processing was performed for 3 minutes. In this way, a phosphor paste containing the CASN red phosphor and the silicone resin was prepared. The phosphor paste thus obtained was dropped into a frame having a height of approximately 140 µm using a dispenser (model: ML-5000 XII, manufactured by Musashi Engineering, Inc.). Then, the phosphor paste was cured by heating in the atmosphere at 150°C for 2 hours. In this way, a resin fluorescent film having a thickness of approximately 130 µm (CASN red phosphor sheet: 10 mm long * 10 mm wide) was formed, and the first wavelength converter was obtained.

### (Fabrication of second wavelength converter)

Similarly to the first wavelength converter, 0.40 g of a LGO near-infrared phosphor and a silicone resin (1 g of KER-2600A, 1 g of KER-2600B) were mixed using a stirring defoaming device, and then further defoamed. In this way, a phosphor paste containing the LGO near-infrared phosphor and the silicone resin was prepared. The phosphor paste thus obtained was dropped into a frame having a height of approximately 320 µm using the dispenser. Then, the phosphor paste was cured by heating in the atmosphere at 150°C for 2 hours. In this way, a resin fluorescent film having a thickness of approximately 300 µm (LGO near-infrared phosphor sheet: 10 mm long * 10 mm wade) was formed, and the second wavelength converter was obtained.

### (Fabrication of near-infrared light emitting device)

A phosphor sheet was arranged on a principal light extraction surface of a blue LED such that the blue LED faces the second wavelength converter of the laminated wavelength converters, and accordingly the near-infrared light emitting device was fabricated.

Light emission characteristics of the obtained near-infrared light emitting device were evaluated. When a current of 10 mA (2.6 V) was applied to a blue LED chip, blue light as primary light was radiated from the blue LED chip. Then, a part of the primary light was converted into a near-infrared fluorescence component by the second wavelength converter. In addition, a part of the primary light passed through the second wavelength converter, was converted into red light as a visible fluorescence component by the first wavelength converter. Then, the near-infrared light emitting device emitted, as output light, mixed light of the blue light as the primary light, the near-infrared fluorescence component, and the visible fluorescence component. For reference, the appearance of the output light was violet light, and the light had a color tone which may not be regarded as white light. The violet light is regarded as light produced by additive color mixing of a blue light component of the primary light, and red light components of the near-infrared fluorescence component and the visible fluorescence component.

FIG. 2 shows the spectral distribution of the output light emitted from the near-infrared light emitting device of the present example. As can be seen from FIG. 2, the spectral distribution of the output light includes a light component derived from wavelength converted light of a phosphor, and a light component 10A derived from the primary light. The light component derived from wavelength converted light of a phosphor includes a light component 20A derived from a near-infrared phosphor included in the second wavelength converter, and a light component 20B derived from a visible phosphor included in the first wavelength converter.

When the center of gravity of wavelength converted light by a phosphor (that is, light components of a near-infrared fluorescence component 20A and a visible fluorescence component 20B, excluding the light component 10A derived from the primary light) of the spectral distribution was examined, the center of gravity was at a wavelength of 813 nm, as shown in FIG. 3. In addition, a spectral intensity in a wavelength range of 660 nm or more and less than 900 nm had the minimum value in the vicinity of a wavelength of 710 nm, and the maximum value in the vicinity of a wavelength of 719 nm (or in the vicinity of a wavelength of 835 nm), and in the spectral distribution, the minimum value was 54% of the maximum value.

As can be seen from FIG. 3, in the spectral distribution, a light intensity gradually decreases as a wavelength becomes shorter from a wavelength of 600 nm, and the light intensity gradually decreases as a wavelength becomes longer from a wavelength of 980 nm.

As can be seen from FIG. 3, according to the present example, by using the LGO phosphor as a near-infrared phosphor and the CASN red phosphor as a visible phosphor in combination, it was possible to provide the near-infrared light emitting device which emits the output light having the spectral distribution and which is suitable for evaluating the determination factor of the market value of objects to be inspected such as fruits and vegetables, for example. Specifically, the spectral distribution of the output light had a light component derived from fluorescent light of a phosphor at least in a wavelength range of 600 nm or more and less than 980 nm, and the center of gravity of the spectral distribution of wavelength converted light by a phosphor was in a wavelength range of 700 nm or more and less than 900 nm. Further, a spectral intensity in a wavelength range of 660 nm or more and less than 900 nm had the minimum value which was more than 50% of the maximum value.

### (Example 2)

A near-infrared light emitting device (wavelength conversion type light emitting element) according to Example 2, which includes a solid-state light emitting element and a wavelength converter as shown in FIG. 5, was configured using the following members 1) to 5). The solid-state light emitting element was a blue laser diode (actual peak wavelength: 453 nm), and the wavelength converter was a resin fluorescent film including three types of phosphors which are the CASN red phosphor, the GGG near-infrared phosphor, and the GSO near-infrared phosphor.
1) Blue laser diode (actual peak wavelength: 453 nm) (manufactured by OSRAM Opto Semiconductors GmbH)
2) CASN red phosphor (Mitsubishi Chemical Corporation)
3) GGG near-infrared phosphor (self-produced product)
4) GSO near-infrared phosphor (self-produced product)
5) Thermosetting silicone resin (product name: KJR-9032, Shin-Etsu Chemical Co., Ltd.)

The wavelength converter and the near-infrared light emitting device were fabricated as follows.

First, a wavelength converter formed of three types of phosphors and a silicone resin was fabricated by means of the following procedures. 4 g of a silicone resin, 0.600 g of a CASN red phosphor, 2.76 g of a GGG near-infrared phosphor, and 8.57 g of a GSO near-infrared phosphor were put into a cylindrical plastic container. Then, these were mixed using a planetary centrifugal mixer to prepare a phosphor paste. The phosphor paste was applied on a wheel substrate (outer diameter: 65 mm, material: MIRO-SILVER) using a screen printer. Thereafter, a heat treatment was performed at 150°C using a curing furnace to fabricate a phosphor wheel in which the volume ratio (vol%) of silicone resin: CASN : GGG : GSO was 58.6 : 4.4 : 8.7 : 28.3. The thickness of the fluorescent film was 45 µm.

The phosphor wheel fabricated in this way was rotated, a laser diode was driven, and the film was irradiated with laser light (primary light) under the following conditions.
1) Rotational speed of phosphor wheel: 10800 rpm
2) LD driving condition: 90 V - 2.0 A
3) Power for irradiating fluorescent film with laser light: 54 W
4) Irradiation spot size diameter: Φ 1.5 mm (design value)

The fluorescent film of the phosphor wheel (wavelength converter) absorbed a part of the laser light, and it became a wavelength-converted near-infrared fluorescence component and visible fluorescence component. Then, laser light which has not been absorbed by the wavelength converter was reflected, and the light was emitted from the near-infrared light emitting device as output light, together with the wavelength-converted near-infrared fluorescence component and visible fluorescence component. Similarly to Example 1, the appearance of the output light was violet light, and the light had a color tone which may not be regarded as white light.

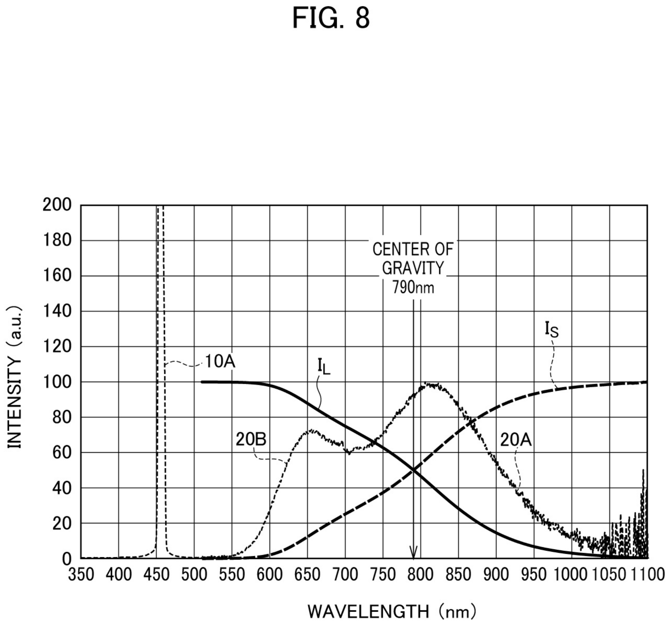

FIG. 8 shows the spectral distribution of the output light emitted from the near-infrared light emitting device of Example 2. As can be seen from FIG. 8, the spectral distribution of the output light includes a light component derived from wavelength converted light of a phosphor (that is, the near-infrared fluorescence component 20A and visible fluorescence component 20B derived from the near-infrared phosphor and visible phosphor included in the wavelength converter), and the light component 10A derived from the primary light.

When the center of gravity of wavelength converted light by a phosphor (that is, light components of the near-infrared fluorescence component 20A and visible fluorescence component 20B, excluding the light component 10A derived from the primary light) of the spectral distribution was examined, the center of gravity was at a wavelength of 790 nm, as shown in FIG. 8. In addition, a spectral intensity in a wavelength range of 660 nm or more and less than 900 nm had the minimum value in the vicinity of a wavelength of 900 nm, and the maximum value in the vicinity of a wavelength of 805 nm, and in the spectral distribution, the minimum value was 52% of the maximum value.

As can be seen from FIG. 8, in the spectral distribution, a light intensity gradually decreases as a wavelength substantially becomes shorter from a wavelength of 600 nm, and the light intensity gradually decreases as a wavelength becomes longer from a wavelength of 980 nm, considering noise.

In the near-infrared light emitting device of the present example, the GGG near-infrared phosphor and GSO near-infrared phosphor as near-infrared phosphors, and the CASN red phosphor as a visible phosphor were used in combination. As can be seen from FIG. 8, according to the present example, it was possible to provide the near-infrared light emitting device which emits the output light having the spectral distribution and which is suitable for evaluating the determination factor of the market value of objects to be inspected such as fruits and vegetables, for example. Specifically, the spectral distribution of the output light had a light component derived from fluorescent light of a phosphor at least in a wavelength range of 600 nm or more and less than 980 nm, and the center of gravity of the spectral distribution of wavelength converted light by a phosphor was in a wavelength range of 700 nm or more and less than 900 nm. Further, a spectral intensity in a wavelength range of 660 nm or more and less than 900 nm had the minimum value which was more than 50% of the maximum value.

In this way, it was possible to configure the near-infrared light emitting device which is suitable for evaluating the determination factor of the market value of objects to be inspected such as fruits and vegetables.

### (Supplementary notes)

By the above description of the embodiments, the following techniques are disclosed.

(Technique 1) A near-infrared light emitting device that emits output light, including: a solid-state light emitting element; and a wavelength converter that includes a phosphor, in which a spectral distribution of the output light has a light component derived from fluorescent light of the phosphor at least in a wavelength range of 600 nm or more and less than 980 nm, a center of gravity of a spectral distribution of wavelength converted light by the phosphor is in a wavelength range of 700 nm or more and less than 900 nm, the center of gravity is a wavelength at which, an integrated value obtained by integrating a light intensity of a spectral distribution in increments of 1 nm of the wavelength converted light from a wavelength of 525 nm to a longer wavelength side, and an integrated value obtained by integrating the light intensity from a wavelength of 1100 nm to a shorter wavelength side, are equal, a spectral intensity in a wavelength range of 660 nm or more and less than 900 nm has a minimum value that is more than 50% of a maximum value, and in the spectral distribution, the light intensity of the spectral distribution of the wavelength converted light gradually decreases as a wavelength becomes shorter from a wavelength of 600 nm.

Due to the configuration, light components are concentrated in red light components and near-infrared components in a wavelength range of 600 nm or more and less than 980 nm. Moreover, light components with relatively small intensity change are output. Therefore, the near-infrared light emitting device can emit the output light suitable for evaluating the decay, sugar content, acidity, ripeness, and water content of fruits and vegetables, for example. Accordingly, the near-infrared light emitting device is suitable for evaluating the determination factor of the market value of objects to be inspected.

(Technique 2) The near-infrared light emitting device according to Technique 1, in which, in the spectral distribution, the light intensity gradually decreases as the wavelength becomes longer from a wavelength of 980 nm. Due to the configuration, the output light has the spectral distribution in which light components with intensities at or above a certain level are uniformly concentrated in red light components and near-infrared components having a wavelength of 980 nm or less. Therefore, the light emitting device can emit the output light including the red light components and near-infrared components with high efficiency.

(Technique 3) The near-infrared light emitting device according to Technique 1 or 2, in which a light component having a spectral distribution in a wavelength range of 600 nm or more and less than 980 nm is derived from electron energy transition of at least one fluorescent ion of an Eu ion and a Cr ion. By using an orthodox Eu ion-activated red phosphor and Cr ion-activated near-infrared phosphor, it is possible to obtain the output light with small intensity change, in which light components are concentrated in red light components and near-infrared components in a wavelength range of 600 nm or more and less than 980 nm. Therefore, the near-infrared light emitting device is suitable for industrial production.

(Technique 4) The near-infrared light emitting device according to any one of Techniques 1 to 3, in which the output light passes through a lens and is output, and the lens collimates the wavelength converted light radiated from the wavelength converter. Due to the configuration, the light radiated from the wavelength converter as diffuse light is converted into collimated light (parallel light) and then is output. Therefore, it is possible to evaluate an object to be inspected by irradiating the object to be inspected with near-infrared light which is emitted from the near-infrared light emitting device that is located far from the object to be inspected without bringing the near-infrared light emitting device in close proximity to the object to be inspected.

(Technique 5) The near-infrared light emitting device according to Technique 4, in which a light entering surface of the lens is arranged to cover an entirety of a light emission surface of the wavelength converter. Due to the configuration, a lot of wavelength converted light enters the light entering surface of the lens. Therefore, the near-infrared light emitting device is suitable for converting the light emitted from the wavelength converter into the collimated light.

(Technique 6) The near-infrared light emitting device according to any one of Techniques 1 to 5, in which the solid-state light emitting element emits primary light, and the primary light is used to irradiate the wavelength converter that rotates. Due to the configuration, a position of an area of the wavelength converter irradiated with the primary light changes according to change in time. Therefore, the wavelength converter of which the temperature rises due to being irradiated with the primary light moves to a position where the wavelength converter is not irradiated with the primary light, and the wavelength converter is cooled down during the time. Therefore, even if the wavelength converter is irradiated with primary light with a high energy density, the temperature rise of the wavelength converter is suppressed, and the near-infrared light emitting device can emit high output light.

(Technique 7) The near-infrared light emitting device according to any one of Techniques 1 to 6, in which the wavelength converter has a plate shape, and a thickness of the wavelength converter is 50 µm or more and less than 500 µm. Due to the configuration, since the wavelength converter is thin, the ratio of wavelength converted light leaked from a side surface of the wavelength converter is reduced, and the ratio of light entering the light entering surface of the lens increases. Therefore, the near-infrared light emitting device can output high-quality coherent light.

(Technique 8) A spectroscopic apparatus that irradiates an object to be inspected with the output light emitted by the near-infrared light emitting device, the spectroscopic apparatus including: the near-infrared light emitting device according to any one of Techniques 1 to 7. Due to the configuration, light components are concentrated in red light components and near-infrared components in a wavelength range of 600 nm or more and less than 980 nm. Moreover, light components with relatively small intensity change are used to irradiate an object to be irradiated. Therefore, the spectroscopic apparatus is suitable for evaluating the decay, sugar content, acidity, ripeness, and water content of fruits and vegetables, for example.

(Technique 9) The spectroscopic apparatus according to Technique 8, in which the spectroscopic apparatus is an inspection apparatus. Due to the configuration, the spectroscopic apparatus irradiates an object to be inspected with light suitable for precisely evaluating the object to be inspected. Therefore, the spectroscopic apparatus is advantageous for the precise inspections for determining the market value of the object to be inspected.

(Technique 10) A spectroscopy using the near-infrared light emitting device according to any one of Techniques 1 to 7. Due to the configuration, light components are concentrated in red light components and near-infrared components in a wavelength range of 600 nm or more and less than 980 nm. Moreover, light components with relatively small intensity change are used to irradiate an object to be irradiated. Therefore, the spectroscopy is suitable for evaluating the decay, sugar content, acidity, ripeness, and water content of fruits and vegetables, for example.

The entire contents of Japanese Patent Application No. 2023-010889 (filed on: January 27, 2023) are incorporated herein by reference.

Although the embodiments herein have been described above, the embodiments herein are not limited to the descriptions thereof, and various modifications are possible within the scope of the gist of the embodiments herein.

### INDUSTRIAL APPLICABILITY

According to the present disclosure, it is possible to provide a near-infrared light emitting device, a spectroscopic apparatus, and a spectroscopy which are suitable for evaluating the determination factor of the market value of an object to be inspected.

### REFERENCE SIGNS LIST

- 1: Solid-state light emitting element
- 2: Wavelength converter
- 3: Lens
- 5: Object to be inspected
- 10: Primary light
- 20: Output light
- 100: Near-infrared light emitting device
- 200: Spectroscopic apparatus

## Claims

1. A near-infrared light emitting device that emits output light, comprising:
a solid-state light emitting element; and
a wavelength converter that includes a phosphor, wherein
a spectral distribution of the output light has a light component derived from fluorescent light of the phosphor at least in a wavelength range of 600 nm or more and less than 980 nm,
a center of gravity of a spectral distribution of wavelength converted light by the phosphor is in a wavelength range of 700 nm or more and less than 900 nm,
the center of gravity is a wavelength at which, an integrated value obtained by integrating a light intensity of a spectral distribution in increments of 1 nm of the wavelength converted light from a wavelength of 525 nm to a longer wavelength side, and an integrated value obtained by integrating the light intensity from a wavelength of 1100 nm to a shorter wavelength side, are equal,
a spectral intensity in a wavelength range of 660 nm or more and less than 900 nm has a minimum value that is more than 50% of a maximum value, and
in the spectral distribution, the light intensity of the spectral distribution of the wavelength converted light gradually decreases as a wavelength becomes shorter from a wavelength of 600 nm.

2. The near-infrared light emitting device according to claim 1, wherein
in the spectral distribution, the light intensity gradually decreases as the wavelength becomes longer from a wavelength of 980 nm.

3. The near-infrared light emitting device according to claim 1 or 2, wherein
a light component having a spectral distribution in a wavelength range of 600 nm or more and less than 980 nm is derived from electron energy transition of at least one fluorescent ion of an Eu ion and a Cr ion.

4. The near-infrared light emitting device according to any one of claims 1 to 3, wherein
the output light passes through a lens and is output, and the lens collimates the wavelength converted light radiated from the wavelength converter.

5. The near-infrared light emitting device according to claim 4, wherein
a light entering surface of the lens is arranged to cover an entirety of a light emission surface of the wavelength converter.

6. The near-infrared light emitting device according to any one of claims 1 to 5, wherein
the solid-state light emitting element emits primary light, and the primary light is used to irradiate the wavelength converter that rotates.

7. The near-infrared light emitting device according to any one of claims 1 to 6, wherein
the wavelength converter has a plate shape, and a thickness of the wavelength converter is 50 µm or more and less than 500 µm.

8. A spectroscopic apparatus that irradiates an object to be inspected with the output light emitted by the near-infrared light emitting device, the spectroscopic apparatus comprising:
the near-infrared light emitting device according to any one of claims 1 to 7.

9. The spectroscopic apparatus according to claim 8, wherein
the spectroscopic apparatus is an inspection apparatus.

10. A spectroscopy using the near-infrared light emitting device according to any one of claims 1 to 7.
